# EUROPEAN PATENT APPLICATION

(11) **EP 4 800 728 A1**
(43) Date of publication of application: **02.09.2026**
(21) Application number: 26159150.7
(22) Date of filing: 17.02.2026
(51) Int. Cl.: H01H 9/18

(54) **STATUS INDICATING SWITCH FOR ELECTRICAL EQUIPMENT, ESPECIALLY MEDICAL EQUIPMENT**

(30) Priority: 28.02.2025 US 202563764612 P
(71) Applicant: Karl Storz SE & Co. KG, 78532 Tuttlingen (DE)
(72) Inventor: DEGOEDE, Grant, Goleta, 93117 (US); DE VISSER, Tim, Goleta, 93117 (US); FELIX, Timothy, Goleta, 93117 (US)

(57) **Abstract**

An exemplary aspect is generally directed toward a state-changing status/power switch. Even more specifically, one exemplary aspect is generally directed toward an illuminated state-changing status switch that provides context regarding an operational state of an associated piece of equipment, such as a medical device. The context can be provided via one or more of an illumination state of the switch, text on the switch and/or icons on the switch. Alternatively or in addition, the switch can include color-changing capabilities with, for example, white or green being an operational state, orange or yellow reflecting a state between power-on and power-off, and white being a powered-down or standby state.

## Description

### FIELD

The present application relates to electronic switches, more specifically, the present application relates to status indicating electrical switches. Even more specifically, the present application relates generally to status indicating electrical switches for electrical equipment, including medical equipment, where knowing the operational status of the equipment can be critical.

### BACKGROUND

Modern medical devices are oftentimes operated or controlled by electronic equipment, which, among other electronic components, comprises electronic switches for example for turning the equipment on and off, selecting operating modes or indicating a processing status. For example, for endoscopic or exoscopic imaging systems, an electronic camera/imaging unit may be used to capture image data, process image signals and display images on monitors. The electronic equipment may serve as an interface between a user and the medical devices, wherein the electronic switches are used to facilitate operation of the medical device according to the user's needs. However, one challenge that exists is to provide a clear and simple to understand communication between the electronic equipment and the user.

### SUMMARY

The present disclosure is generally directed to an improved electronic context-conveying switch and a medical device comprising an electronic context-conveying switch as defined in independent claim 1. Embodiments of the electronic context-conveying switch are disclosed in the depending claims.

The electronic context-conveying switch according to the present disclosure comprises a controller, a touch-sensitive circuit, and a display portion. The display portion is configured to present context information regarding an operational state of an associated electronic device via one or more of color, an icon and a message.

One aspect is generally directed toward an electronic context-conveying switch in form of a state-changing status/power switch/button. Even more specifically, one exemplary aspect is generally directed toward an illuminated state-changing status switch that provides context regarding an operational state of an associated piece of equipment, such as a medical device. The context can be provided via one or more of an illumination state of the switch, text on the switch and/or icons on the switch. Alternatively, or in addition, the switch can include color-changing capabilities with, as just one example, white or green being an operational state, orange or yellow reflecting a state between power-on and power-off, and white being a powered-down or standby state.

In accordance with one aspect, the exemplary switch is equipped with a plurality of LEDs that are color-changing and/or of different colors.

Alternatively, or in addition, the exemplary switch can include a display, such as a Liquid Crystal Display (LCD), a Light Emitting Diode (LED) display, an Organic LED (OLED) display, a Twisted Nematic (TN) display, an In-Plane Switching (IPS) display, a Vertical Alignment (VA) display, a Mini-LED display, a quantum dot display, a QD-OLED, a Micro-LED, and/or the like.

Alternatively, or in addition, the switch can be a push-button, touch sensitive, capacitive/resistive touch, and/or the like. In some embodiments the switch may include haptic feedback indicating that the button has been activated, such as the presence of a snap dome.

Generally, capacitive touch switches are configured to detect touch, for example of a finger(s), or proximity, of a finger(s), by measuring changes in capacitance occurring at the switch. When a user touches the surface of a capacitive touch switch, or comes within a specified range, the capacitance of the switch changes, and this detectable change triggers a proximity event which in turn caused a change in state of the switch and the associated electronic device.

Alternatively, or in addition, the switch can optionally be combined with LCD (Liquid Crystal Display) and/or LED (Light Emitting Diode) touch screen(s) that operates in a similar fashion, with one major difference being the backlighting technology utilized in each: LCD screens use Cold Cathode Fluorescent Lamps (CCFLs) for backlighting and LED screens use light-emitting diodes for backlighting. Both are capable of having either capacitive or resistive touch sensitive technologies that determine where a touch occurs on the screen to cause/trigger an underlying function.

One of the challenges medical professionals have is knowing in real-time what is the operational status of a piece of equipment. In many medical facilities, equipment is moved from one location to another and is used for different patients by different medical personnel at different times. However, many of the pieces of equipment, for various reasons including information security, network access, order of operation boot-up, etc., need to complete a specific start-up or shut-down sequence to avoid damage to the system, to be able to save or upload patient data, to run required checks, to initialize system elements or configurations, and/or the like. Since medical professionals are typically under time constraints, having improved context and insight into the operational state of a piece of equipment can improve the overall work environment, prevent improper use of equipment, and give personnel, for example personnel performing an operating room turn-over procedure, valuable insight in order to avoid mistakes which might occur due to severe time pressure, such as unplugging a machine before its shut-down sequence has been completed.

Alternatively, or in addition, timing information can be provided via the switch either numerically, and/or through color changes, that convey how long before the equipment is expected to be in its next state. For example, and in conjunction with any of the other features, the switch could display a message, such as "ready in 90 seconds" indicating that an initialization sequence will be complete in 90 seconds and the device will be ready for use. This could occur with a color change of one or more portions of the switch from, for example, white morphing to green, and/or a numerical count down.

Similarly, during operation, the switch could turn orange after a press indicting the switch in a shut-down procedure with a counter counting down from 60 seconds until the equipment has completed the powering off procedure. This could be combined with a color change morphing from green to white, or the like.

Alternatively, or in addition, the switch can include one or more color changing portions, such as glass or plastic rings, that can be used to convey state information such as the various states described herein and discussed above. These ring(s) can optionally have a status bar that moves in a clockwise or counterclockwise fashion indicating the portion of the shut-down, start-up, or other operation that is complete. For example, if the ring is illuminated from the 12 o'clock position, clockwise, to the 10 o'clock position, then that would represent that the system is approximately 80% complete with whatever the underlying function of the equipment would be. The illuminated portion of the light can also indicate the underlying process. For example a boot-up of the system could proceed in filling the annular indicator in a clockwise fashion in a green color, while a red colored annular progression could indicate that the system is shutting down, and at which point in the shut down the system presently exists.

While the exemplary disclosure will be described in relation to certain equipment states and corresponding information displayed/shown/conveyed by the switch, it is to be appreciated that the switch's operation can be modified to account for any one or more states of the associated equipment, and the type, color, messaging, and/or operation of the switch programmed to accommodate and reflect those various states to provide the user with equipment operational state context information.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together.

The term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

The term "computer-readable medium" or "memory" as used herein refers to any tangible storage and/or transmission medium that participate in providing instructions to a processor for execution. Such a medium may take many forms, including but not limited to, non-volatile media, volatile media, and transmission media. Non-volatile media includes, for example, NVRAM, or magnetic or optical disks. Volatile media includes dynamic memory, such as main memory. Common forms of computer-readable media include, for example, a floppy disk, a flexible disk, hard disk, magnetic tape, or any other magnetic medium, magneto-optical medium, a CD-ROM, any other optical medium, punch cards, paper tape, any other physical medium with patterns of holes, a RAM, a PROM, and EPROM, a FLASH-EPROM, a solid state medium like a memory card, any other memory chip or cartridge, a carrier wave as described hereinafter, or any other medium from which a computer can read. A digital file attachment to e-mail or other self-contained information archive or set of archives is considered a distribution medium equivalent to a tangible storage medium. When the computer-readable media is configured as a database, it is to be understood that the database may be any type of database, such as relational, hierarchical, object-oriented, and/or the like. Accordingly, the disclosure is considered to include a tangible storage medium or distribution medium and prior art-recognized equivalents and successor media, in which the software implementations of the present disclosure are stored.

The preceding is a simplified summary of the disclosure to provide an understanding of some aspects of the disclosure. This summary is neither an extensive nor exhaustive overview of the disclosure and its various aspects, embodiments, and/or configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and/or configurations of the disclosure are possible utilizing, alone or in combination, one or more of the features set forth above or described in detail below.

These and other features and advantages of this technology are described in, or are apparent from, the following detailed description of the exemplary embodiments.

### BRIEF DESCRIPTION OF THE DRAWINGS

Exemplary embodiments will be described in detail, with reference to the following figures, wherein:
FIG. 1 illustrates an exemplary environmental view of a switch according to one aspect.
FIG. 2 illustrates another exemplary switch according to one aspect.
FIG. 3 illustrates another exemplary illuminated switch according to one aspect.
FIG. 4 illustrates another exemplary perspective view of switch with a display according to one aspect.
FIG. 5 illustrates another exemplary perspective view of switch with a display according to one aspect.
FIG. 6 illustrates an exemplary state diagram of a switch according to one aspect.

### DETAILED DESCRIPTION

The exemplary embodiments of this technology will be described in relation to a context-conveying switch for electronic equipment.

For purposes of explanation, numerous details are set forth in order to provide a thorough understanding of the present technological solution. It should be appreciated however that the presently disclosed technology may be practiced in a variety of ways beyond the specific details set forth herein. Furthermore, while the exemplary embodiments illustrated herein show various components of this system collocated, it is to be appreciated that the various components of the system can be located at distant portions of a distributed or local network or circuit.

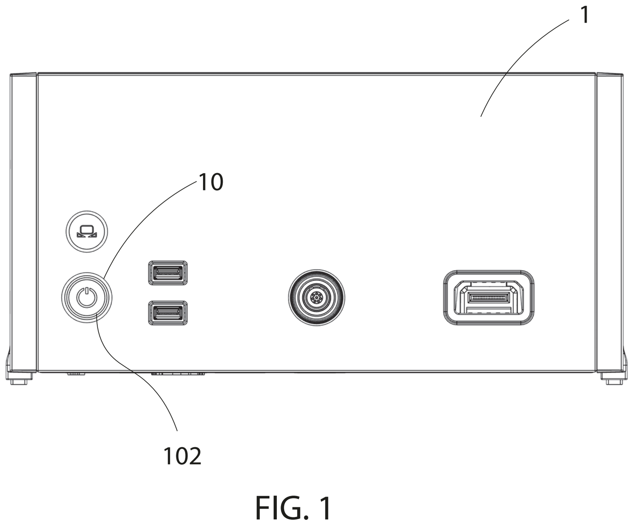

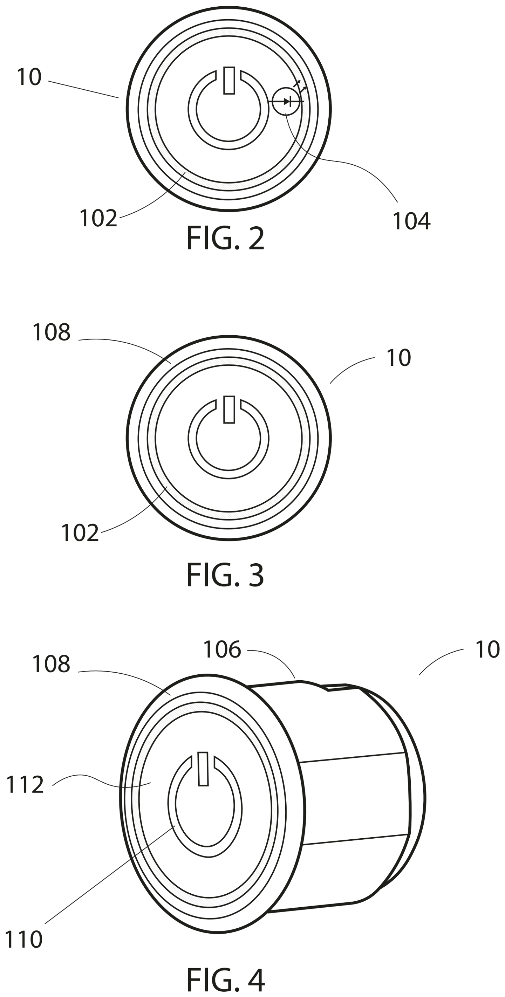

FIG. 1 illustrates an exemplary environmental view of a switch 10 according to one aspect. In FIG. 1, the switch 10 functions as the power button for the electronic device/equipment 1, and in this example is shown as an element of a camera control system (CCU), frequently used in conjunction with endoscopic and exoscopic procedures, wherein cameras connected to endoscopes, video endoscopes, or exoscopes may be connected to the CCU. In this example, the switch 10 includes a color-changing glowing ring 102 (that can be made of plastic, glass, or any other suitable material known in the art, that is back lit with, for example, one or more LEDs (as indicated in FIG. 2)) that may be shown, illustratively, as green, for example, indicating normal operation of the device 1. As discussed, the switch 10 can be a touch-sensitive switch such as a capacitive or resistive touch sensitive switch. Alternatively, the switch can include a mechanical portion to achieve activation, and may include haptic feedback, indicating to the user, that the function of the button has been, or is being, activated. One potential embodiment might include a snap dome as an element of a sealed button assembly, to be discussed further below. As discussed hereinafter, the device 1 can have a plurality of operational states with the particular glow/illumination status of the switch ring 102 corresponding to that operational state or a transition between operational states. Additionally, the device 1 could also optionally include a key (for example printed on a decal or silk-screened thereon) that shows the various device states and their corresponding indications for quick reference by a user.

While FIG. 1 shows the switch 10 illustratively installed in the lower-left corner, it is to be appreciated the switch can be located anywhere on device 1 where it is both physically and visually accessible. Moreover, the switch can be accompanied by other controls/ports such as a white-level calibration interface, USB ports, and connections, as in this example. In use, a user can touch with their finger to switch states of the device 1, such as from off to on, from on to off, and to/from other states depending on the number of operational states of the device 1. Additionally, the switch activation surface may include a capacitive sensor configured to register information not only on the activation of the switch, but on the identity of the operator pressing it. For example, the switch could be configured to read a fingerprint of the presser, or a key could be used to activate the switch, the key having a specific identifier associated therewith, allowing the device 1 to obtain information about the activator or activation element. For example, the identifier could indicate to the device 1 a desired configuration or for the system and any auxiliary devices, such as endoscopic cameras attached thereto. For example, a user, when entering a clinical room, might press the switch with a thumb, the switch registers the user's login credentials, boots the device 1 and sets the device and its peripherals to match the customized configuration preferred by the user. In more sterile environments, such as operating rooms, most or all of the medical personnel may be wearing surgical gloves, which may hinder the reading of identifying biometric indicators, such as fingerprints. In such cases, another form of identification, such as an RFID implanted identification card or glove, that may be placed in contact with the switch in order to activate the device, change its state, preload customized configurations, etc. Of course, some biometric identifiers, such as identifying vein patterns in a user's finger, may be determinable through gloves, and such means are compatible with various embodiments presented herein. Additionally, the intensity of the glow/illumination of the ring 102 could be user adjustable with the assistance of a controller discussed hereinafter. The color or a pattern of colors indicating the state, or independent of the state indicator, could provide a simple visual confirmation to the ultimate user of the customization. For example, if surgical assistant A, activates the device with a key card associated with surgeon B, instead of surgeon C, who is performing the procedure, surgeon C can easily identify, upon entering the operating theater, that the device 1 is not set to her customized preferences, as her customizations are indicated by a blue color at the switch, and the current customization is yellow. Moreover, the switch 10 could include additional functionality associated with longer touches/holds, again depending on the different states/functions of the device 1. As one example, if the device 1 is a critical piece of medical equipment, the switch 10 could require a 3 second press before starting a shut down sequence to prevent accidental shut downs.

FIG. 2 illustrates another exemplary switch according to one aspect. The switch 10 includes one or more LEDs 104 that are configured to illuminate the ring 102 which can be seen glowing, for example, green. As discussed, the intensity, color, flashing, flashing sequence, and/or status of the LED(s) can be programmable and/or user adjustable. For example, to indicate status, the switch could include graduations akin to a fuel gauge, and when the device 1 is transitioning from an operational state to an off state, the "fuel gauge" "empties" until the device shuts down - thereby conveying to the user how close the device is to being in the "off" state. Moreover, while FIG. 3 illustratively shows a ring 102, the illuminated portion of the switch need not be a ring but could be any shape, such as the form of a power icon 110. The ring or other icon may be any size within the constraints of the switch 10 surface and/or may occupy any one or more portions of the switch 10, including the bezel 108. Additionally, or alternatively, the switch can be sealed and include one or more stainless steel portions, such as a stainless steel bezel.

FIG. 4 illustrates another exemplary perspective view of switch with a display according to one aspect. FIG. 4 illustrates in greater detail the bezel 108, the casing 106, and the screen 112, with a displayed icon 110. In this illustrative embodiment, the switch 10 has a display 112, such as an illuminated touch screen, that can be configured not only for status-conveying color changes, but also may optionally include icons representing what underlying function the is being performed and/or is being indicated by the color displayed. For example, the icon 110 could be a power icon, and the color indicate the state, for example: green (power on), red (malfunction), orange (powering down), yellow (powering up) or white (plugged in but not powered on). Other icons could indicate other operational states relevant thereto. For example, an hourglass icon could indicate the passage of time or a timer countdown.

As will be appreciated, since the screen 112, in some embodiments, has display capabilities and can be a touch sensitive screen, a touch screen, an infrared touch screen, a screen adapted for use with a stylus, or the like, any displayable information could be presented thereon.

Optionally, the screen 112 could be programmed with one or more animations again to assist the user in understanding the operational state of the device 1. The screen could also show a percentage complete of a certain function.

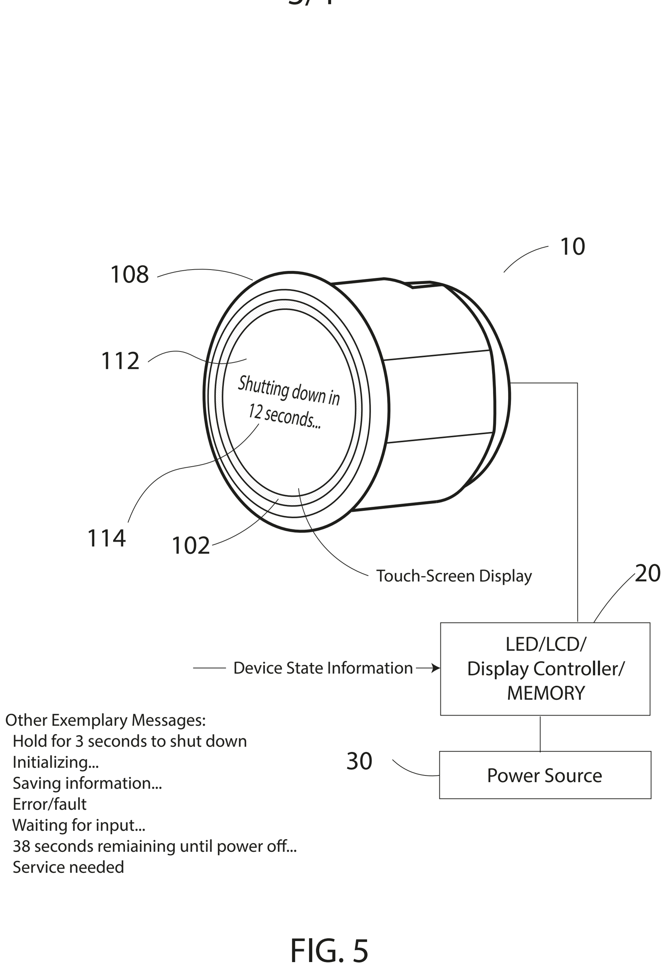

FIG. 5 illustrates another exemplary perspective view of switch with a display according to one aspect. Specifically, FIG. 5 shows the display 112 with a message 114. The messaging 114 can be used with any of the other functionality discussed herein to convey state information to a user. Additionally, and again since this embodiment of the switch includes a display, any message(s) can be programmed to be displayed in any color/font/intensity/language. As shown in the example of FIG. 5, the text message may also accompany a glowing ring that may be part of the display 112 (e.g. pixels on a circular display dedicated to displaying a ring), or it may be in addition to the display (e.g. a back-lit LED ring surrounding a, for example, LCD, e-ink, OLED, etc. display).

The switch can be controlled by a controller 20. The controller is configured to receive state information and convert that sate information into a control signal that governs what is displayed on the switch 10. The controller 20 is powered by a power source 30, and further includes a memory that stores information as to which information should be stored based on the input state information. The controller 20 may optionally be part of a device 1, such as a camera control unit.

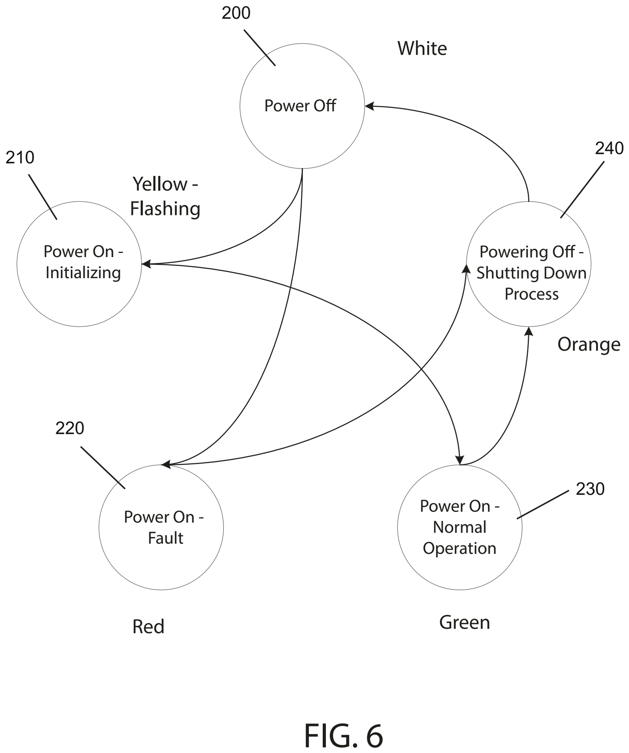

FIG. 6 illustrates an exemplary state diagram of a switch according to one aspect. The various exemplary states correlate to what information is shown on/by the switch and when. As is appreciated, the state diagram and corresponding states are dependent on the functionality of the underlying device, and there can be more or less states. For one simple exemplary device, the state diagram can include a power off state 200, a power-on initializing state 210, a power-on fault state 220, a power-on normal operation state 230 and a powering-off, shutting down process state 240. Transition to/from the various states is shown in the figure and the corresponding displayed information for the screen shown adjacent to the state. In the show example, the power off state 200 is indicated by a white color, the power-on initializing state 210 is indicated by a yellow flashing light, the power-on fault state 220 is indicated by a red color, the power-on normal operation state 230 is indicated by a green color and the powering-off, shutting down process state 240 is indicated by an orange color. Of course, other color codes may be used characterize the states of the state diagram.

The systems, methods and protocols of this disclosure can be implemented on a special purpose computer, a programmed microprocessor or microcontroller and peripheral integrated circuit element(s), an ASIC or other integrated circuit, a digital signal processor, a flashable device, a hard-wired electronic or logic circuit such as discrete element circuit, a programmable logic device such as PLD, PLA, FPGA, PAL, an image processing device, an endoscope, a medical imaging device, or the like. In general, any device (or one or more equivalent means) capable of implementing a state machine that is in turn capable of implementing the methodology illustrated herein can be used to implement the various methods, protocols and techniques disclosed herein.

Furthermore, the disclosed methods may be readily implemented in software stored on a non-transitory computer-readable information storage media using, for example, object or object-oriented software development environments that provide portable source code that can be used on a variety of computer or workstation platforms. Alternatively, the disclosed system may be implemented partially or fully in hardware using standard logic circuits or VLSI design. Whether software or hardware is used to implement the systems in accordance with this disclosed technology is dependent on the speed and/or efficiency requirements of the system, the particular function, and the particular software or hardware systems or microprocessor or microcomputer systems being utilized. The systems, methods and protocols illustrated herein can be readily implemented in hardware and/or software using any known or later developed systems or structures, devices and/or software by those of ordinary skill in the applicable art from the functional description provided herein and with a general basic knowledge of the computer, medical, optical and/or endoscope arts.

Moreover, the disclosed methods may be readily implemented in software that can be stored on a computer-readable storage medium, executed on programmed general-purpose computer with the cooperation of a controller and memory, a special purpose computer, a microprocessor, or the like. The systems and methods of this technology can be implemented as a program embedded on personal computer such as an applet, JAVA^{®} or CGI script, as a resource residing on a server or computer workstation, as a routine embedded in a dedicated system or system component, or the like. The system can also be implemented by physically incorporating the system and/or method into a software and/or hardware system, such as the hardware and software systems of an imaging/medical/electronic device.

While this description is described in terms of exemplary embodiments, it should be appreciated that individual aspects of the technology could be separately claimed and one or more of the features of the various embodiments can be combined.

While the exemplary embodiments illustrated herein discuss the various components collocated, it is to be appreciated that the various components of the system can be located a distant portions of a distributed network, such as a telecommunications network and/or the Internet or within a dedicated communications network. Thus, it should be appreciated that the components of the system can be combined into one or more devices or collocated on a particular node of a distributed network, such as a communications network. As will be appreciated from the following description, and for reasons of computational efficiency, the components of the system can be arranged at any location within the distributed network without affecting the operation of the system.

Although the present disclosure describes components and functions implemented in the aspects, embodiments, and/or configurations with reference to particular standards and protocols, the aspects, embodiments, and/or configurations are not limited to such standards and protocols. Other similar standards and protocols not mentioned herein are in existence and are considered to be included in the present disclosure. Moreover, the standards and protocols mentioned herein and other similar standards and protocols not mentioned herein are periodically superseded by faster or more effective equivalents having essentially the same functions. Such replacement standards and protocols having the same functions are considered equivalents included in the present disclosure.

The present disclosure, in various aspects, embodiments, and/or configurations, includes components, methods, processes, systems and/or apparatus substantially as depicted and described herein, including various aspects, embodiments, configurations embodiments, subcombinations, and/or subsets thereof. Those of skill in the art will understand how to make and use the disclosed aspects, embodiments, and/or configurations after understanding the present disclosure. The present disclosure, in various aspects, embodiments, and/or configurations, includes providing devices and processes in the absence of items not depicted and/or described herein or in various aspects, embodiments, and/or configurations hereof, including in the absence of such items as may have been used in previous devices or processes, e.g., for improving performance, achieving ease and\or reducing cost of implementation.

The foregoing discussion has been presented for purposes of illustration and description. The foregoing is not intended to limit the disclosure to the form or forms disclosed herein. In the foregoing Detailed Description for example, various features of the disclosure are grouped together in one or more aspects, embodiments, and/or configurations for the purpose of streamlining the disclosure. The features of the aspects, embodiments, and/or configurations of the disclosure may be combined in alternate aspects, embodiments, and/or configurations other than those discussed above. This method of disclosure is not to be interpreted as reflecting an intention that the claims require more features than are expressly recited in each claim. Rather, as the following claims reflect, inventive aspects lie in less than all features of a single foregoing disclosed aspect, embodiment, and/or configuration. Thus, the following claims are hereby incorporated into this Detailed Description, with each claim standing on its own as a separate preferred embodiment of the disclosure.

Moreover, though the description has included description of one or more aspects, embodiments, and/or configurations and certain variations and modifications, other variations, combinations, and modifications are within the scope of the disclosure, e.g., as may be within the skill and knowledge of those in the art, after understanding the present disclosure. It is intended to obtain rights which include alternative aspects, embodiments, and/or configurations to the extent permitted, including alternate, interchangeable and/or equivalent structures, functions, ranges or steps to those claimed, whether or not such alternate, interchangeable and/or equivalent structures, functions, ranges or steps are disclosed herein, and without intending to publicly dedicate any patentable subject matter.

An exemplary aspect of the disclosure is directed toward an electronic context-conveying switch comprising: a controller, a touch sensitive circuit, and a display portion; the display portion configured to present context information regarding an operational state of an associated electronic device via one or more of color, an icon and a message.

Further exemplary aspects may refer to: a switch, wherein the display portion is a touch-sensitive display. Any of the above aspects, wherein the touch-sensitive display is a LED-based display. Any of the above aspects, further comprising memory configured to store state information for the associated electronic device and a corresponding one or more of color, an icon and a message to be displayed. Any of the above aspects, wherein the display portion is a touch-sensitive LED display. Any of the above aspects, further comprising a bezel, a power source and a case. Any of the above aspects, wherein the display portion is a plastic or glass ring. Any of the above aspects, further comprising one or more icons. Any of the above aspects, wherein the switch is hermetically sealed. Any of the above aspects, wherein the switch is configured to change an operational state of the associated electronic device based on a finger touch. Any of the above aspects, wherein the finger touch includes a long press or several presses. Any of the above aspects, further comprising one or more LEDs. Any of the above aspects, further comprising one or more dimmable LEDs. Any of the above aspects, further comprising one or more color changing LEDs. Any of the above aspects, wherein the associated electronic device is a medical device. Any of the above aspects, wherein the switch is programmed to reflect the status of a plurality of operational states of the associated electronic device. Any of the above aspects, wherein an intensity of a light emitted by the switch is one or more of programmable and adjustable. Any of the above aspects, wherein the context relates to a specific state in a state diagram of the associated electronic device. Any of the above aspects, further comprising one or more of an LED, LCD, and touch-screen display. Any of the above aspects, further comprising a haptic feedback generator configured to provide haptic feedback to the operating when the switch is activated. Any of the above aspects, wherein the haptic feedback generator comprises a snap dome.

A further exemplary aspect may refer to: a medical device comprising, a first camera input, configured to receive video data from a first camera; and an electronic, context-conveying switch comprising: a controller; a touch sensitive circuit; and a display portion, the display portion configured to present context information regarding an operational state of an associated electronic device via one or more of color, an icon and a message.

Any one or more of the aspects/embodiments as substantially disclosed herein optionally in combination with any one or more other aspects/embodiments as substantially disclosed herein. One or more means adapted to perform any one or more of the above aspects/embodiments as substantially disclosed herein. Any one of the aspects/features/embodiments in combination with any one or more other aspects/features/embodiments.

Use of any one or more of the aspects or features as disclosed herein.

It is therefore apparent that there has been provided, in accordance with the present disclosure, systems and methods for a status switch. While this disclosure has been described in conjunction with a number of embodiments, it is evident that many alternatives, modifications and variations would be or are apparent to those of ordinary skill in the applicable arts. Accordingly, this disclosure is intended to embrace all such alternatives, modifications, equivalents and variations that are within the spirit and scope of the disclosed technology.

### LISTING OF REFERENCE NUMBERS

1 - device / equipment
10 - switch
20 - controller
30 - power source
102 - ring
104 - LED(s)
106 - casing
108 - bezel
110 - icon / power icon
112 - display / screen
114 - message / messaging area
200 - power off state
210 - power-on initializing state
220 - power-on fault state
230 - power-on normal operation state
240 - powering-off, shutting down process state

## Claims

1. Electronic context-conveying switch (10) comprising: a controller (20); a touch-sensitive circuit; and a display portion (112), the display portion (112) configured to present context information regarding an operational state of an associated electronic device (1) via one or more of color, an icon (110) and a message (114).

2. Switch (10) according to claim 1, wherein the display portion (112) is a touch-sensitive display.

3. Switch (10) according to claim 2, wherein the touch-sensitive display is an LED (104) based display.

4. Switch (10) according to any of the above claims, further comprising memory configured to store state information for the associated electronic device (1) and a corresponding one or more of color, an icon (110) and a message (114) to be displayed.

5. Switch (10) according to any of the above claims, further comprising a power source (30) and a case (106), and, optionally, a bezel (108).

6. Switch (10) according to any of the above claims, wherein the display portion (112) includes a plastic or glass ring (102).

7. Switch (10) according to any of the above claims, wherein the switch (10) is configured to change an operational state of the associated electronic device (1) based on a finger touch.

8. Switch (10) according to any of the above claims, further comprising one or more color-changing LEDs (104).

9. Switch (10) according to any of the above claims, wherein the associated electronic device (1) is a medical device.

10. Switch (10) according to any of the above claims, wherein the switch (10) is programmed to reflect the status of a plurality of operational states of the associated electronic device (1).

11. Switch (10) according to any of the above claims, wherein an intensity of a light emitted by the switch (10) is one or more of programmable and adjustable.

12. Switch (10) according to any of the above claims, wherein the context relates to a specific state in a state diagram of the associated electronic device (1).

13. Switch (10) according to any of the above claims, further comprising one or more of an LED (104), LCD, and touch-screen display.

14. Switch (10) according to any of the above claims, further comprising a haptic feedback generator configured to provide haptic feedback to the operator when the switch (10) is activated.

15. Medical device (1) comprising a camera input, configured to receive video data from a camera; and a switch (10) according to any of the above claims.
